# FASCICULE DE BREVET EUROPEEN

(11) **EP 1 422 288 B1**
(45) Date de publication et mention de la délivrance du brevet: **03.01.2007**
(21) Numéro de dépôt: 03292518.2
(22) Date de dépôt: 10.10.2003
(51) Int. Cl.: C11D 17/00, C11D 3/22, C11D 10/04, A61Q 1/14, A61Q 19/10, A61Q 5/02

(54) **Composition liquide de nettoyage à base de savons et de tensio-actifs synthétiques;utilisations pour le nettoyage des matières kératiniques humaines**
Flüssige Reinigungsmittel auf Basis von Seifen und synthetischen Tensiden; Ihre Verwendung zur Reinigung von menschlichen Keratinmaterialien
Liquid cleaning composition containing soaps and synthetic surfactants; its use for cleaning human keratinous materials

(30) Priorité: 21.11.2002 FR 0214598
(43) Date de publication de la demande: 26.05.2004
(73) Titulaire: L'ORÉAL, 75008 Paris (FR)
(72) Inventeur: Ribery, Delphine, 92300 Levallois Perret (FR); Bissey, Beugras, Laure, 92300 Levallois-Perret (FR)
(74) Mandataire: Miszputen, Laurent

(56) Documents cités:
- EP-A- 0 133 345
- WO-A-97/27279
- US-A- 4 387 040
- US-A- 5 910 472
- DATABASE WPI Section Ch, Week 198832 Derwent Publications Ltd., London, GB; Class A97, AN 1988-225105 XP002245797 & JP 63 161083 A (KAO CORP), 4 juillet 1988 (1988-07-04)

## Description

La présente invention a trait à une composition liquide de nettoyage à base de savons et de tensioactif synthétiques, caractérisée par le fait qu'elle contient dans un milieu aqueux cosmétiquement acceptable
(a) au moins un acide gras ou un mélange d'acides gras totalement neutralisés dans une quantité inférieure ou égale à 20% en poids par rapport au poids total de la composition ; ledit acide gras ou lesdits acides gras étant neutralisés par une base minérale choisie parmi les hydroxydes de métaux alcalins ou l'hydroxyde d'ammonium ou par une base organique choisie parmi les amines et les alcanolamines ;
(b) au moins un tensioactif anionique synthétique ;
(c) au moins un tensioactif amphotère ; la quantité totale en tensioactif anionique synthétique et en tensioactif amphotère étant inférieure ou égale à 10% en poids en matière active par rapport au poids total de la composition ;
(d) au moins un épaississant du type composé cellulosique non-ionique.

La présente invention concerne également ses utilisations dans les domaines cosmétique ou dermatologique, notamment comme produits de nettoyage des matières kératiniques humaines et plus particulièrement de la peau.

On connaît actuellement dans le domaine des produits pour la douche les savons traditionnels sous forme de pain solide et les "savons liquides " sous forme de lait, de crème ou de gel crème conditionnées en général en tube, en flacon, en flacon pompe, en aérosol douche mousse ou en flacon-pompe mousse.

Les savons solides traditionnels sont constitués de sels d'acide gras de métal alcalin ont un bon pouvoir nettoyant, produisent une mousse dense, crémeuse et aérée et s'éliminent très bien au lavage en laissant à l'utilisateur après rinçage à l'eau une sensation de crissant rapidement perçue sur la peau. Cependant, ces produits présentent de nombreux inconvénients. Leur utilisation est peu hygiénique. Ils vieillissent mal. Leur transport n'est pas pratique. De plus, ils laissent la peau rêche, rugueuse et tiraillée après application et lavage.

Les "savons liquides" sont apparus sur le marché de l'hygiène corporelle dans les années 1940 ; ils contenaient à cette époque des savons d'acide gras de potassium. D'autres formules lavantes liquides à base de savons ont été décrites dans les brevets US 4,387,040 ; US 4,310,432 et US 310,433.

Dans les années 1980, d'autres types de formulations lavantes se sont développées . Ces nouvelles formulations comprennent quatre grandes familles de formulation détergente : (1) celles à base de lauryl sulfate ; celles à base d'alpha-oléfine sulfonate, (3) celles à base de mélange de tensio-actifs synthétiques anioniques, amphotères et/ou non-ioniques ; (4) les formulations mixtes à bases de savons et de tensio-actifs synthétiques.

Ces compositions de nettoyage pour le corps ou les mains selon le conditionnement doivent présenter une consistance et une rhéologie appropriées pour une bonne appréhension du produit par l'utilisateur et un bon étalement sur la surface de la peau à laver. Elles contiennent en général un système épaississant choisis par exemple parmi les électrolytes comme le chlorure de sodium, le chlorure de potassium ou le sulfate de potassium ; les alcanolamides comme le cocamide DEA ou le cocamide MEA ; les esters de polyethylèneglycol et de monoacide ou diacide stéarique comme le distéarate de polyéthylèneglycol 6000 ou leurs mélanges. ,

Ces compositions ont été décrites notamment dans les documents suivants :
- Formulation Technology of Liquid Soaps by Eugene M. Franck - Cosmetic & Toiletries Vol 97, 1982, pages 49-54.
- Liquid Soap, a challenge for the formulator by Roger Hart - Household & Personal Care Products Industry May 1981, pages 46-47 ;
- Comparison of Detergent Based versus Soap Based Liquid Soaps by Dennis W. Dyer and Thomas Hassapis- Soap/Cosmetics/Chemical Specialities for July , 1993pages 36; 38 and 40 ;
- The Evolution of Liquid Soap by Larry Lundmark - Cosmetic & Toiletries Vol 107, December 1992, pages 49-52.

Les formulations liquides pour la douche présentent par rapport aux savons solides divers avantages :
- Elles sont plus hygiéniques par leur conditionnement,
- Elles sont plus faciles à transporter par leur conditionnement ;
- leur utilisation est plus simple ;
- Elles laissent la peau plus douce et moins tiraillée après application et rinçage.

Cependant, des tests consommateurs ont montré que les produits d'hygiène corporelle liquides avaient tendance à laisser après rinçage une sensation glissante et l'impression que du produit reste sur la peau.

Un des souhaits des consommateurs est donc de disposer de produits pour le lavage du corps sous forme liquide apportant à la fois les avantages obtenus avec les formulations liquides actuellement sur le marché et ceux obtenus avec les savons solides. On cherche ainsi à obtenir une formulation liquide qui, à la sortie du conditionnement, possède un bon pouvoir détergent, produit une mousse qui démarre rapidement, qui est crémeuse, dense et aérée et qui s'élimine très rapidement au rinçage en laissant après rinçage une sensation de crissant rapidement perçue sur la peau.

On connaît dans l'état de la technique des formulations liquides à base de savons, de tensio-actif anionique et de tensio-actif amphotère. Dans le document "Formulation Technology of Liquid Soaps by Eugene M. Franck - Cosmetic & Toiletries Vol 97, 1982, pages 49-54", l'exemple 13 décrit une composition à base de savon (ie : potassium de cocoate), du sel de sodium de lauryl sulfate et de cocoamidopropylbétaine. Cette formulation est épaissie par le mélange cocamide MEA et polyéthyleneglycol 6000 distéarate. Dans le document W097/27279, on connaît également des formulations à base de savons, de tensio-actif anionique du type lauryl éther sulfate et d'un tensio-actif amphotère du type bétaine épaissies par le cocamide DEA. Les qualités de mousse et /ou d'élimination au rinçage obtenues par ces formulations ne sont pas pleinement satisfaisantes.

On connaît également dans le brevet US 5,910,472 des compositions détergentes conditionnantes à base de tensioactifs hydrosolubles (anionique, amphotère, zwitterionique, non-ionique ou des mélanges), d'une cellulose non-ionique modifiée hydrophobe et d'un polymère cationique conditionneur. Ce document ne préconise pas des formulations à base de tensioactifs détergents synthétiques et de savons d'acides gras détergents. L'exemple VI contient du cocoamphodiacétate et du Sodium Laureth-3-Sulfate et du stéarate de zinc.

On connaît également la demande JP 63-161083 décrivant des formulations liquides détergentes comprenant l'association d'une hydroxypropylcellulose particulière et de tensioactifs fortement électrolytiques L'association tensioactifs synthétiques et savons détergents n'est pas préconisée.

La demanderesse a découvert de manière surprenante qu'en utilisant (1) un système détergent constitué de savons dans une quantité déterminée et d'un mélange de tensio-actif anionique et de tensio-actif amphotère dans une quantité déterminée et (2) au moins un épaississant du type composé cellulosique non-ionique, on obtenait une formulation lavante sous forme liquide ayant à la fois les avantages obtenus avec les formulations liquides actuellement sur le marché et ceux obtenus avec les savons solides à savoir : une formulation liquide qui présente un bon pouvoir nettoyant, produisant une mousse qui démarre rapidement, qui est crémeuse, dense et aérée et qui s'élimine très rapidement au rinçage en laissant après rinçage une sensation de crissant rapidement perçue sur la peau ainsi qu'une peau douce et non tiraillée.

La présente invention a donc pour objet une composition de nettoyage liquide à base de savons et de tensioactifs synthétiques, caractérisée par le fait qu'elle contient dans un milieu aqueux cosmétiquement acceptable :
(a) au moins un acide gras ou un mélange d'acides gras totalement neutralisés dans une quantité inférieure ou égale à 20% en poids par rapport au poids total de la composition ; ledit acide gras ou lesdits acides gras étant neutralisés par une base minérale choisie parmi les hydroxydes de métaux alcalins ou l'hydroxyde d'ammonium ou par une base organique choisie parmi les amines et les alcanolamines ;
(b) au moins un tensioactif anionique ;
(c) au moins un tensio-actif amphotère ; la quantité totale en tensio-actif anionique et en tensio-actif amphotère étant inférieure ou égale à 10% en poids par rapport au poids total de la composition ;
(d) au moins un épaississant du type composé cellulosique non-ionique.

Un autre objet de l'invention consiste en l'utilisation cosmétique de la composition telle que définie ci-dessus, comme produits de nettoyage et/ou de démaquillage des matières kératiniques.

Un autre objet de l'invention consiste en un procédé cosmétique de nettoyage des résidus de salissure des matières kératiniques humaines caractérisé par le fait qu'on applique la composition de l'invention, sur lesdites matières kératiniques, en présence d'eau, qu'on masse pour former une mousse et qu'on élimine la mousse formée et les résidus de salissure par rinçage à l'eau.

Dans toute la description, on entend par « matières kératiniques humaines » la peau (corps ou visage y compris les mains, les lèvres, les paupières et le cuir chevelu), les ongles et les phanères comme les cheveux, les cils et les sourcils.

Dans toute la description, on entend par « liquide», toute composition se présentant sous forme de solution épaissie, lait, crème, gel, gel-crème.

Lorsque les compositions ne sont pas conditionnée sous forme de flacon-pompe ou d'aérosol, leur viscosité à 25°C mesurée au Brookfield DV-II (de chez Chimilab Essor) avec le mobile RV 3, une vitesse de 5 rpm et un temps de rotation du mobile de 30 sec varie en général de 50 à 20000. mPa.s et plus particulièrement de 1000 à 12000 mPa.s.

Lorsque les compositions sont conditionnées sous forme de flacon-pompe ou d'aérosol, leur viscosité à 25°C mesurée au Brookfield DV-II (de chez Chimilab Essor) avec le mobile RV 3, une vitesse de 5 rpm et un temps de rotation du mobile de 30 sec varie en général de 50 à 20000. mPa.s et plus particulièrement de 1000 à 12000 mPa.s.

Par composé cellulosique, on entend selon l'invention tout composé polysaccharidique possédant dans sa structure des enchaînements de résidus glucose unis par des liaisons β-1,4.

Les acides gras conformes à l'invention sont en général des acides gras, comportant une chaîne alkyle linéaire ou ramifiée, saturée ou insaturée, ayant de 6 à 30 atomes de carbone et de préférence 12 à 22 atomes de carbone.

Comme acide gras, on peut citer notamment l'acide laurique, l'acide myristique, l'acide palmitique et l'acide stéarique et leurs mélanges. Et plus particulièrement un mélange d'acide laurique, d'acide myristique, d'acide palmitique et d'acide stéarique.

Les bases utilisées pour neutraliser totalement ces acides carboxyliques sont les bases minérales comme les hydroxydes de métaux alcalins (soude et potasse), aussi les hydroxydes de métaux ou l'ammoniaque ou encore les bases organiques comme la triéthanolamine, monéthanolamine, monoisopropanolmaine, la N-méthylglucamine, la lysine et l'arginine. On choisira plus particulièrement la potasse.

L'acide gras ou le mélange d'acides gras sont généralement introduits dans la composition sous forme libre puis on ajoute ensuite la base, la neutralisation se faisant in situ.

Le ou les acides gras conformes à l'invention sont présents dans des quantités ne dépassant pas 20% en poids par rapport au poids de la composition. Au delà de cette concentration, d'une part la mousse obtenue est peu aérée et d'autre part les savons précipitent fortement dans le support aqueux et donne aux consommateurs l'impression désagréable d'avoir un dépôt blanc sur la peau.

Le ou les acides gras conformes à l'invention sont présents de préférence dans des quantités allant de 1 à 20% en poids et plus préférentiellement de 5 à % 10 % en poids par rapport au poids total de la composition.

Comme tensioactifs anioniques utilisables dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les α-oléfine-sulfonates, les paraffine-sulfonates ; les alkylsulfosuccinates, les alkyléthersulfosuccinates, les alkylamide-sulfosuccinates ; les alkylsulfoacétates ; les acylsarcosinates ; et les acylglutamates, les groupes alkyle et acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone et leurs mélanges.

En outre, on peut encore citer les acides d'alkyl-D-galactoside uroniques , les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés, en particulier ceux comportant de 2 à 50 groupements oxyde d'éthylène ; et leurs sels de métaux alcalins, d'ammonium, d'amines, d'aminoalcoôls ou de métaux alcalino-terreux.

On utilisera de préférence les sels d'alkyléthersulfates en C₆-C₂₄ comportant de 1 à 30 groupements oxyde d'éthylène , en particulier les sels de métaux alcalins ou alcalino-terreux, d'ammonium, d'amine ou d'aminoalcool et plus particulièrement les sels de sodium et encore plus particulièrement les alkyl (C₁₂-C₁₄)éthersulfates de sodium oxyéthylénés comportant un nombre moyen de groupes oxyde d'éthylène compris entre 1 et 4 et plus particulièrement le Sodium Laureth Sulfate (nom CTFA) tel que le produit commercial vendu sous le nom TEXAPON AOS 225 UP, TEXAPON N702, TEXAPON NSW commercialisé par la société COGNIS ou EMPICOL ESB3/FL2, EMPICOL ESB3/FL3, EMPICOL ESB70/FL2 commercialisé par la société HUNTSMAN.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes; et leurs mélanges.

Parmi les dérivés d'amines, on peut citer les produits commercialisés sous la dénomination MIRANOL®, tels que décrits dans les brevets US-2 528 378 et US-2 781 354 et classés dans le dictionnaire CTFA, 3ème édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO-) (1)

dans laquelle :
Rₐ représente un groupe alkyle dérivé d'un acide Ra-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(C) (2)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Ra' représente un groupe alkyle d'un acide R_{a'}-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique. A titre d'exemple, on peut citer le cocoamphodiacétate commercialisé sous la dénomination commerciale MIRANOL® C2M concentré par la société RHODIA.

On utilisera, de préférence, les alkyl(C₈-C₂₀)bétaïnes et plus particulièrement le cocobétaine telle que les produits commerciaux MIRATAINE BB/FLA de RHODIA ou EMPIGEN BB/FL de Huntsman.

Selon la présente invention, la quantité totale de tensioactif anionique et de tensioactif amphotère ne doit pas dépasser 10% en poids en matière active par rapport au poids total de la composition. En effet, au delà de cette quantité, la sensation de crissant perçue sur la peau est sensiblement altérée.

Le ou les tensioactifs anioniques synthétiques conformes à l'invention sont de préférence présents dans des quantités allant de 0,1 à 10% en poids en matière active et plus préférentiellement de 0,5 à 5 % en poids par rapport au poids total de la composition.

Le ou les tensioactifs amphotères conformes à l'invention sont de préférence présents dans des quantités allant de 0,1 à 10% en poids en matière active et plus préférentiellement de 0,5 à 5 % en poids par rapport au poids total de la composition.

De manière préférentielle, on utilisera le ou les savons et le tensioactif anionique dans un rapport en poids savons/tensioactif anionique synthétique supérieur à 4 :1 afin d'améliorer l'élimination au rinçage.

Selon l'une des caractéristiques essentielles de l'invention, la composition de l'invention comprend au moins un épaississant du type composé cellulosique non-ionique.

Les composés cellulosiques de l'invention sont choisis de préférence parmi les éthers de cellulose non-ioniques. Parmi les éthers de cellulose non ioniques, on peut citer les alkylcelluloses telles que les méthylcelluloses et les éthylcelluloses ; les hydroxyalkylcelluloses telles que les hydroxyméthylcelluloses, les hydroxyéthylcelluloses et les hydroxypropylcelluloses ; les celluloses mixtes hydroxyalkyl-alkylcelluloses telles que les hydroxypropyl-méthylcelluloses, les hydroxyéthyl-méthylcelluloses, les hydroxyéthyl-éthylcelluloses et les hydroxybutyl-méthylcelluloses et les hydroxyalkylcelluloses modifiées par une chaîne alkyl.

On utilisera plus particulièrement une hydroxypropylméthylcellulose comme les produits commerciaux METHOCEL E, F, J ou K vendus par Dow Coming et encore plus particulièrement le METHOCEL E 4MQG ou le METHOCEL F 4M.

Le ou les composés cellulosiques non-ioniques conformes à l'invention représentent de préférence de 0,01 à 3% en poids en matière active et encore plus préférentiellement de 0,05 à 1.5% en poids par rapport au poids total de la composition.

Les compositions conformes à l'invention peuvent comporter en plus un ou plusieurs épaississants additionnels tels que :
- les électrolytes comme le chlorure de sodium, le chlorure de potassium ou le sulfate de potassium ;
- les alcanolamides comme le cocamide DEA ou le cocamide MEA ;
- les esters de polyethylèneglycol et de monoacide ou diacide stéarique comme le distéarate de polyéthylèneglycol 6000 ou leurs mélanges.
- les biopolymères polysaccharidiques comme la gomme de xanthane, la gomme de guar, les alginates ;
- les polymères synthétiques tels que les polyacryliques comme le CARBOPOL 980, le CARBOPOL 1382 commercialisé par la société NOVEON, les copolymères acrylate/acrylonitrile tels que le HYPAN SS201 commercialisé par la société KINGSTON ;
- les argiles telles que les smectites, les hectorites modifiées ou non telles que les produits BENTONE commercialisés par la société RHEOX, les produits LAPONITE commercialisés par la société SOUTHERN CLAY PRODUCTS, le produit VEEGUM HS commercialisé par la société R.T. VANDERBILT ;
- leurs mélanges.

Les agents épaississants additionnels, sont de préférence présents dans des concentrations préférentielles allant de 0,05 à 4% et plus particulièrement de 0,1 à 3% en poids au poids total de la composition.

On utilisera de préférence comme système épaississant un mélange d'hydroxypropylmethylcellulose et d'alcanolamide et en particulier un mélange hydroxypropylmethylcellulose/cocamide MEA et plus particulièrement un mélange comprenant au moins une hydroxypropylméthylcellulose, au moins un alcanolamide et au moins un électrolyte et encore plus particulièrement un mélange hydroxypropylméthylcellulose/cocamide MEA/chlorure de potassium.

Les compositions selon l'invention peuvent contenir en plus un ou plusieurs tensioactifs non ioniques. Ce sont des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)-aminopropylmorpholine; et leurs mélanges.

Le milieu aqueux cosmétiquement acceptable des compositions de l'invention peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition. De préférence, la composition comprend de 50 à 95 % en poids d'eau par rapport au poids total de la composition.

Les compositions de l'invention contiennent de préférence en plus un ou plusieurs polymères cationiques du type polyquaternium, qui apportent douceur et onctuosité à la composition moussante.

Ces polymères cationiques peuvent être choisis de préférence parmi les polymères suivants :
Polyquaternium 5 tel que le produit MERQUAT 5 commercialisé par la société CALGON ;
Polyquaternium 6 tel que le produit SALCARE SC 30 commercialisé par la société CIBA, et le produit MERQUAT 100 commercialisé par la société CALGON ;
Polyquaternium 7 tel que les produits MERQUAT S, MERQUAT 2200 et MERQUAT 550 commercialisés par la société CALGON, et le produit SALCARE SC 10 commercialisé par la société CIBA ;
Polyquaternium 10 tel que le produit Polymer JR400 commercialisé par la société AMERCHOL;
Polyquaternium 11 tel que les produits GAFQUAT 755, GAFQUAT 755N et GAFQUAT 734 commercialisés par la société ISP ;
Polyquaternium 15 tel que le produit ROHAGIT KF 720 F commercialisé par la société ROHM;
Polyquaternium 16 tel que les produits LUVIQUAT FC905, LUVIQUAT FC370, LUVIQUAT HM552 et LUVIQUAT FC550 commercialisés par la société BASF ;
Polyquaternium 22 tel que le produit MERQUAT 280 commercialisé par la société CALGON ;
Polyquaternium 28 tel que le produit STYLEZE CC10 commercialisé par la société ISP ;
Polyquaternium 39 tel que le produit MERQUAT PLUS 3330 commercialisé par la société CALGON ;
Polyquaternium 44 tel que le produit LUVIQUAT CARE commercialisé par la société BASF;
Polyquaternium 46 tel que le produit LUVIQUAT HOLD commercialisé par la société BASF;
Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

En outre, les compositions de l'invention peuvent contenir des adjuvants habituellement utilisés dans les produits cosmétiques pour la toilette. Ils sont choisis notamment parmi les huiles, les actifs, les parfums, les conservateurs, les séquestrants, les agents nacrant ou opacifiant, les pigments, les nacres, les charges minérales ou organiques telles que le talc, le kaolin, les poudres de silice ou de polyéthylène, les colorants solubles.

Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas les propriétés recherchées pour la composition de l'invention.

Comme exemple d'huile, on peut citer les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles. La quantité d'huiles ne doit pas modifier la propriété recherchée pour la composition de l'invention : elle est d'au maximum 15 % en poids et mieux d'au maximum 10 % en poids par rapport au poids total de la composition, et elle va de préférence de 0,1 à 5 % en poids et mieux de 0,1 à 3 % en poids par rapport au poids total de la composition.

Comme actifs, on peut citer par exemple les filtres solaires ; les agents desquamants ; les agents hydratants ; les agents dépigmentants; les pro-pigmentants ; les alpha-hydroxyacides ; les agents antibactériens ; les agents antiradicalaires ; les agents anti-pollution ; les anti-inflammatoires ; les rétinoïdes ; les extraits d'algues, de champignons, de végétaux, de levures, de bactéries ; les protéines hydrolysées, partiellement hydrolysées ou non hydrolysées ; les enzymes, les hormones, les vitamines et leurs dérivés, les flavonoides et isoflavones, et leurs mélanges.

Les compositions conformes à l'invention peuvent notamment contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol, les alcools gras en particulier les alcools stéarylique, cétylique, béhénylique et leurs mélanges.

Les compositions selon l'invention présentent un pH final varie de préférence de 6 à 10 selon l'application choisie. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique. Dans le cadre des gels douches, le pH variera de préférence de 8 à 10.

Les compositions de l'invention peuvent être conditionnées selon l'application choisie sous forme de flacon, de tube, de flacon-pompe, de dispositif aérosol ou tout autre mode de conditionnement approprié pour une composition liquide de lavage.

Les compositions de nettoyage de la présente invention conditionnées dans des dispositifs aérosols peuvent contenir n'importe quel agent propulseur usuellement employé pour la préparation de compositions aérosols. On peut citer en particulier les gaz hydrocarbonés, comme par exemple le propane, le n-butane, l'isobutane et leurs mélanges; les gaz fluorés comme par exemple le chlorodifluorométhane, le dichlorodifluorométhane, le difluoroéthane, le chlorodifluoroéthane, le dichlorotétrafluoroéthane, etc. et leurs mélanges ; les gaz hydrofluorocarbonés ; le dimethyl ether et les mélanges de dimethyl ether avec un ou plusieurs gaz hydrocarbonés ; l'azote, l'air et le dioxyde de carbone et leurs mélanges peuvent également être utilisés comme gaz propulseurs dans la présente invention. De façon préférentielle, on utilise dans la présente invention des gaz hydrocarbonés ayant de 2 à 6 atomes de carbone et plus particulièrement un mélange iso-butane, propane, n-butane. Le ou les gaz propulseurs sont présents dans le dispositif à raison de 0,1 à 15% en poids et plus préférentiellement de 1 à 8% en poids par rapport au poids total de la composition.

Un autre objet de l'invention consiste en l'utilisation cosmétique de la composition telle que définie ci-dessus, comme produit de nettoyage et/ou de démaquillage des matières kératiniques humaines et plus particulièrement de la peau.

Les compositions de l'invention peuvent notamment être utilisées comme produit pour la douche ; produit pour le bain ; comme produit de nettoyage des mains ; comme shampooing ; comme produit démaquillant des yeux (pas de test de tolérance de fait pour les yeux) et/ou du visage.

Un autre objet de l'invention consiste en un procédé cosmétique de nettoyage des résidus de salissure des matières kératiniques humaines et plus particulièrement de la peau, caractérisé par le fait qu'on applique sur lesdites matières kératiniques la composition de l'invention, en présence d'eau, qu'on masse pour former une mousse et qu'on élimine la mousse formée et les résidus de salissure par rinçage à l'eau.

Les exemples qui suivent servent à illustrer l'invention sans toutefois présenter un caractère limitatif. Les quantités indiquées sont en % en poids sauf mention contraire, et les noms des composés en noms chimiques ou noms CTFA.

| **Matières premières (%MA)** | **Exemple 1** | **Exemple 2** |
|---|---|---|
| | | |
| | **(%en poids en matière active)** | **(%en poids en matière active)** |
| Acide laurique | 5,00 | 5,00 |
| Acide myristique | 0,86 | 0,86 |
| Acide stéarique | 0,79 | 0,79 |
| Acide palmitique | 1,68 | 1,68 |
| Hydroxyde de potassium | 2,14 | 2,21 |
| Laureth sulfate de sodium | 1,02 | 2,00 |
| Cocobétaine | 4,86 | 3,00 |
| Glycérine | 4,00 | 2,00 |
| Chlorure de potassium | 2,50 | - |
| Chlorure de sodium | 1,05 | 2,30 |
| Cocamide MEA | 0,50 | 1,00 |
| Hydroxypropylméthylcellulose | 0,25 | 0,25 |
| DMDM hydantoine | 0,11 | 0,11 |
| Sequestrant | qs | qs |
| Parfum | qs | qs |
| Colorants | qs | qs |
| Eau | 75,25 | 77,70 |

### Les exemples 1 à 2 selon l'invention sont préparées selon le mode opératoire suivant :

On hydrate l'hydroxypropylmethylcellulose dans l'eau froide, on agite pendant 15 à 20 minutes. On ajoute ensuite le chlorure de sodium, le sequestrant, la glycérine et le laureth sulfate de sodium toujours sous agitation. On ajoute ensuite le cocamide MEA, l'acide laurique, l'acide myristique, l'acide stéarique et l'acide palmitique. On homogénéise. On introduit l'hydroxyde de potassium et on homogénéise. On chauffe le mélange à 75°C sous agitation. Le mélange ainsi obtenu est homogène, incolore et limpide. On refroidit le mélange à 30-35°C et on ajoute le parfum, le conservateur DMDM Hydantoin, homogénéise. On introduit alors le cocobétaïne et homogénéise. On ajuste aux normes de viscosité si nécessaire avec le chlorure de sodium.

### Performances sensorielles :

Les qualités de mousse et de rinçage des compositions des exemples 1 et 2 ont été évaluées par un panel de 7 personnes selon le protocole décrit ci-dessous :

Avant toute utilisation des produits, les mains sont lavées au savon puis convenablement rincées.

Puis les étapes suivantes sont effectués :
- mouiller les mains en les passant sous l'eau courante thermostatée à 38°C avec un débit de 8 I/mn,
- placer 2 g de produit dans le creux de l'une des mains,
- ajouter 3 ml de la même eau et travailler le produit pendant 15 secondes,
- évaluer les critères de qualités de mousse du test,
- rincer les mains sous la même eau courante pendant 10 secondes avec une évaluation du rinçage à 5 secondes et à 10 secondes.

### Les critères suivants sont évalués et sont notés sur une échelle de 0 à 10.

Démarrage de la mousse : la note attribuée est d'autant plus élevée que le démarrage est plus rapide.

La quantité : pouvoir moussant ; la note attribuée est d'autant plus élevée que le produit a développé plus de mousse dans les mains

La sensation de crémeux : la note attribuée est d'autant plus élevée que la sensation crémeuse est grande.

Elimination au rinçage: sensation de crissant 5 et 10 secondes après le rinçage.

Les résultats moyens sensoriels pour chacun des critères sont présentés dans le tableau suivant :

| **Qualité de la mousse** | **Ex. 1** | **Ex. 2** |
|---|---|---|
| Démarrage de la mousse | 6 | 6.1 |
| Quantité (pouvoir moussant) | 7,2 | 7,3 |
| Sensation de crémeux (densité) | 8,1 | 7,7 |
| Elimination au rinçage à 5 s | 4.3 | 5.5 |
| Elimination au rinçage à10 s | 8,8 | 9,1 |

### Exemples comparatives 3 et 4

On a réalisé une formulation lavante A selon l'exemple 13 du document "Formulation Technology of Liquid Soaps by Eugene M. Franck - Cosmetic & Toiletries Vol 97, 1982, pages 49-54". Cette composition est à base de savon (ie : cocoate de potassium), de sel de sodium de lauryl éther sulfate et de cocoamidopropylbétaine. Elle est épaissie par le mélange cocamide MEA et polyéthyleneglycol 6000 distéarate

On a également réalisé une formulation lavante B selon l'exemple 14 du document WO97/27279, à base de savon, de tensio-actif anionique du type lauryl éther sulfate et d'un tensio-actif amphotère du type bétaine épaissie par le cocamide DEA.

| **Matières premières (%MA)** | **Exemple A (hors invention)** | **Exemple B (hors invention)** |
|---|---|---|
| | | |
| | **(%en poids en matière active)** | **(%en poids en matière active)** |
| Acide laurique | 5,00 | 9,00 |
| Acide myristique | 1,15 | 2,05 |
| Acide stéarique | 0,79 | 1,43 |
| Acide palmitique | 1,68 | 3,03 |
| Hydroxyde de potassium | 2,21 | 3,98 |
| Laureth sulfate de sodium | 4,20 | 4,60 |
| Cocobétaine | 5,00 | 4,00 |
| Glycérine | - | 2,00 |
| Chlorure de sodium | 0,92 | 1,87 |
| Cocamide MEA | 4.00 | 3,00 |
| PEG-150 distéarate | 1.50 | 0,25 |
| DMDM hydantoine | 0,11 | 0,11 |
| Sequestrant | qs | qs |
| Parfum | qs | qs |
| Colorants | qs | qs |
| Eau | 76,36 | 65,68 |

Les qualités de mousse et de rinçage des compositions des exemples A et B ont été évaluées par un panel de 7 personnes selon le même protocole décrit ci-dessus:

Les résultats moyens sensoriels pour chacun des critères sont présentés dans le tableau suivant :

| **Qualité de la mousse** | **Ex. 1** | **Ex. 2** | **Ex. A** | **Ex. B** |
|---|---|---|---|---|
| Démarrage de la mousse | 6 | 6.1 | 5,29 | 5,29 |
| Quantité (pouvoir moussant) | 7,2 | 7,3 | 5,86 | 6,86 |
| Sensation de crémeux (densité) | 8,1 | 7,7 | 8,00 | 7,57 |
| Elimination au rinçage à 5s | 4.3 | 5.5 | 4,86 | 3,29 |
| Elimination au rinçage à 10s | 8,8 | 9,1 | 7,57 | 7,29 |

On constate que
- la formulation A de l'art antérieur produit une mousse plus lente au démarrage, moins dense et qui s'élimine plus difficilement au rinçage après 10 s par rapport à celles produites par les compositions 1 et 2 de l'invention.
- la formulation B de l'art antérieur produit une mousse plus lente au démarrage, moins dense et qui s'élimine plus difficilement au rinçage par rapport à celles produites par les compositions 1 et 2 de l'invention

## Revendications

1. Composition de nettoyage liquide à base de savons et de tensioactifs synthétiques, **caractérisée par le fait qu'**elle contient dans un milieu aqueux cosmétiquement acceptable:
(a) au moins un acide gras ou un mélange d'acides gras totalement neutralisés dans une quantité inférieure ou égale à 20% en poids par rapport au poids total de la composition ; ledit acide gras ou lesdits acides gras étant neutralisés par une base minérale choisie parmi les hydroxydes de métaux alcalins, ou l'hydroxyde d'ammonium ou par une base organique choisie parmi les amines et les alcanolamines ;
(b) au moins un tensioactif anionique synthétique ;
(c) au moins un tensio-actif amphotère ; la quantité totale en tensio-actif anionique synthétique et en tensio-actif amphotère étant inférieure ou égale à 10% en poids en matière active par rapport au poids total de la composition ;
(d) au moins un épaississant du type composé cellulosique non-ionique.

2. Composition selon la revendication 1, **caractérisée en ce que** l'acide gras est un acide carboxylique, comportant une chaîne alkyle linéaire ou ramifiée, saturée ou insaturée, ayant de 6 à 30 atomes de carbone et de préférence 12 à 22 atomes de carbone.

3. Composition selon la revendication 2, **caractérisée en ce que** l'acide gras est choisi parmi l'acide laurique, l'acide myristique, l'acide palmitique, l'acide stéarique et leurs mélanges.

4. Composition selon la revendication 3, **caractérisée en ce qu'**elle contient un mélange d'acide gras constitué par l'acide laurique, l'acide myristique, l'acide palmitique et l'acide stéarique.

5. Composition selon l'une quelconque des revendications 1 à 4, où la base est la potasse.

6. Composition selon l'une quelconque des revendications 1 à 5, où le ou les acides gras sont présents dans des quantités allant de 1 à 20% en poids et plus préférentiellement de 5 à % 10 % en poids par rapport au poids total de la composition.

7. Composition selon l'une quelconque des revendications 1 à 6, où le ou les tensioactifs anioniques synthétiques sont choisis parmi les sels des composés suivants :
les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les α-oléfine-sulfonates, les paraffine-sulfonates ; les alkylsulfosuccinates, les alkyléthersulfosuccinates, les alkylamide-sulfosuccinates ; les alkylsulfoacétates ; les acylsarcosinates ; les acylglutamates pour lesquels les groupes alkyles et acyles comportent de 6 à 24 atomes de carbone et le groupe aryle désigne un groupe phényle ou benzyle, les esters d'alkyle en C₆-C₂₄ et d'acides polyglycoside-carboxyliques , les alkylsulfosuccinamates, les acyliséthionates et les N-acyltaurates dont le groupe alkyle ou acyle comportent de 12 à 20 atomes de carbone ; les acyllactylates dont le groupe acyle comporte de 8 à 20 atomes de carbone et leurs mélange.

8. Composition selon l'une quelconque des revendications 1 à 7, où le ou les tensioactifs anioniques synthétiques sont choisis parmi les acides d'alkyl-D-galactoside uroniques ; les acides alkyl(C₆-C₂₄)éther-carboxyliques polyoxyalkylénés , les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels.

9. Composition selon l'une quelconque des revendications 7 et 8, où les sels sont choisis parmi les sels de métaux alcalins, de métaux alcalino-terreux, d'ammonium, d'amine ou d'aminoalcool.

10. Composition selon l'une quelconque des revendications 1 à 9 où le ou les tensio-actif anioniques synthétiques sont choisis parmi les sels d'alkyléthersulfates en C₆-C₂₄ comportant de 1 à 30 groupements oxyde d'éthylène.

11. Composition selon la revendication 9 ou 10, où le tensio-actif anionique synthétique est choisi les alkyl (C₁₂-C₁₄)éthersulfates de sodium ayant un nombre moyen de groupes oxyde d'éthylène compris entre 1 et 4.

12. Composition selon la revendication 11, où le tensio-actif anionique synthétique est le Sodium Laureth Sulfate.

13. Composition selon l'une quelconque des revendications 1 à 12, où le ou les tensio-actif anioniques synthétiques sont présents dans des quantités allant de 0,1 à 10% en poids en matière active et plus préférentiellement de 0,5 à 5 % en poids par rapport au poids total de la composition.

14. Composition selon l'une quelconque des revendications 1 à 13**,** où le tensioactif amphotère est choisi parmi les dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique hydrosolubilisant choisi parmi carboxylate, sulfonate, sulfate, phosphate ou phosphonate ; les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes; et leurs mélanges.

15. Composition selon la revendication 14 où le tensio-actif amphotère est choisi parmi les composés de structures respectives (1) et (2) :
Rₐ-CONHCH₂CH₂-N(R_{b})(R_{c})(CH₂COO-) (1)
dans laquelle :
Rₐ représente un groupe alkyle dérivé d'un acide Ra-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et
Rₐ'-CONHCH₂CH₂-N(B)(C) (2)
dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Ra' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

16. Composition selon la revendication 15, où le tensio-actif amphotère est choisi parmi cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

17. Composition selon la revendication 16, où le ou les tensio-actifs amphotères sont choisis parmi les alkyl(C₈-C₂₀)bétaïnes

18. Composition selon la revendication 17, où le tensio-actif amphotère est la cocobétaïne.

19. Composition selon l'une quelconque des revendications 1 à 18, où le ou les tensio-actifs amphotères sont présents dans des quantités allant de 0,1 à 10% en poids en matière active et plus préférentiellement de 0,5 à 5 % en poids par rapport au poids total de la composition. (à vérifier)

20. Composition selon l'une quelconque des revendications 1 à 19, où le rapport en poids savon(s)/tensioactif anionique est supérieur à 4 :1.

21. Composition selon l'une quelconque des revendications 1 à 20, où les composés cellulosiques sont choisis parmi les éthers de cellulose non-ioniques.

22. Composition selon la revendication 21, où les éthers de cellulose non ioniques, sont choisis parmi les alkylcelluloses ; les hydroxyalkylcelluloses ; les celluloses mixtes hydroxyalkyl-alkylcelluloses.

23. Composition selon la revendication 21 ou 22, où le composé cellulosique non-ionique est une hydroxypropylcellulose.

24. Composition selon l'une quelconque des revendications 1 à 23, où le ou les composés cellulosiques représentent de 0,01 à 3% et encore plus préférentiellement de 0,05 à 1.5 % en poids par rapport au poids total de la composition.

25. Composition selon l'une quelconque des revendications 1 à 24, contenant en plus au moins un autre épaississant additionnel.

26. Composition selon la revendication 25, où les épaississants additionnels sont choisis parmi.
- les électrolytes ;
- les alcanolamides ;
- les esters de polyethylèneglycol et de monoacide ou diacide stéarique ;
- les biopolymères polysaccharidiques ;
- les polymères synthétiques ;
- les argiles ;
- leurs mélanges.

27. Composition selon la revendication **26**, où les épaississants additionnels sont présents dans des concentrations allant de 0,05 à 4 % en poids en matière active par rapport et plus particulièrement de 0,1 à 3% en poids au poids total de la composition.

28. Composition selon l'une quelconque des revendications 25 à 27 dans laquelle le système épaississant est un mélange d'hydroxypropylméthylcellulose et d'alcanolamide.

29. Composition selon la revendication 28, dans laquelle le système épaississant est un mélange hydroxypropylméthylcellulose/cocamide MEA.

30. Composition selon l'une quelconque des revendications 25 à 29, dans laquelle le système épaississant est un mélange contenant une hydroxypropylméthylcellulose, un alcanolamide et un électrolyte.

31. Composition selon la revendication 30, dans laquelle le système épaississant est un mélange hydroxypropylméthylcellulose/cocamide MEA/chlorure de potassium.

32. Composition selon l'une quelconque des revendications 1 à 31, **caractérisée en ce** le milieu aqueux cosmétiquement acceptable comprend au moins, un solvant choisi parmi les alcools inférieurs ; les polyols ; les sucres et leurs mélanges.

33. Composition selon l'une quelconque des revendications 1 à 32, **caractérisée en ce qu'**elle comprend en plus au moins un polymère cationique du type polyquaternium.

34. Composition selon l'une quelconque des revendications 1 à 33, **caractérisée en ce qu'**elle comprend en plus au moins un tensio-actif non-ionique.

35. Composition selon l'une quelconque des revendications 1 à 34, **caractérisée en ce qu'**elle comprend en plus au moins un adjuvant choisi parmi les huiles, les actifs, les parfums, les conservateurs, les séquestrants, les pigments, les nacres, les charges minérales ou organiques, les colorants.

36. Composition selon la revendication 35, où le ou les actifs sont choisis parmi les filtres solaires ; les agents desquamants ; les agents hydratants ; les agents dépigmentants; les pro-pigmentants ; les alpha-hydroxyacides ; les agents antibactériens ; les agents antiradicalaires ; les agents anti-pollution ; les anti-inflammatoires ; les rétinoïdes ; les extraits d'algues, de champignons, de végétaux, de levures, de bactéries ; les protéines hydrolysées, partiellement hydrolysées ou non hydrolysées ; les enzymes, les hormones, les vitamines et leurs dérivés, les flavonoides et isoflavones, et leurs mélanges.

37. Utilisation cosmétique d'une composition telle que définie dans l'une quelconque des revendications 1 à 36**,** comme produit de nettoyage et/ou de démaquillage des matières kératiniques humaines.

38. Utilisation selon la revendication 37, comme produit pour le bain ou la douche.

39. Utilisation selon la revendication 37, comme produit pour le nettoyage des mains.

40. Utilisation selon la revendication 37, comme shampooing.

41. Utilisation selon la revendication 37, comme produit démaquillant des yeux, du visage ou des lèvres.

42. Procédé cosmétique de nettoyage des résidus de salissure des matières kératiniques humaines, **caractérisé par le fait qu'**on applique une composition telle que définie dans l'une quelconque des revendications 1 à 36 sur lesdites matières kératiniques, en présence d'eau, qu'on masse pour former une mousse et qu'on élimine la mousse formée et les résidus de salissure par rinçage à l'eau.

## Claims

1. Liquid cleaning composition based on soaps and on synthetic surfactants, **characterized in that** it comprises, in a cosmetically acceptable aqueous medium:
(a) at least one fatty acid or a mixture of fatty acids which are completely neutralized in an amount of less than or equal to 20% by weight with respect to the total weight of the composition, the said fatty acid or the said fatty acids being neutralized by an inorganic base chosen from alkali metal hydroxides or ammonium hydroxide or by an organic base chosen from amines and alkanolamines;
(b) at least one synthetic anionic surfactant;
(c) at least one amphoteric surfactant, the total amount of synthetic anionic surfactant and of amphoteric surfactant being less than or equal to 10% by weight as active material with respect to the total weight of the composition;
(d) at least one thickener of the nonionic cellulose compound type.

2. Composition according to Claim 1, **characterized in that** the fatty acid is a carboxylic acid comprising a saturated or unsaturated and linear or branched alkyl chain having from 6 to 30 carbon atoms and preferably 12 to 22 carbon atoms.

3. Composition according to Claim 2, **characterized in that** the fatty acid is chosen from lauric acid, myristic acid, palmitic acid, stearic acid and their mixtures.

4. Composition according to Claim 3, **characterized in that** it comprises a fatty acid mixture composed of lauric acid, myristic acid, palmitic acid and stearic acid.

5. Composition according to any one of Claims 1 to 4, where the base is potassium hydroxide.

6. Composition according to any one of Claims 1 to 5, where the fatty acid or acids are present in amounts ranging from 1 to 20% by weight and more preferably from 5 to 10% by weight, with respect to the total weight of the composition.

7. Composition according to any one of Claims 1 to 6, where the synthetic anionic surfactant or surfactants are chosen from the salts of the following compounds:
alkyl sulphates, alkyl ether sulphates, alkylamido ether sulphates, alkylarylpolyether sulphates, monoglyceride sulphates; alkyl sulphonates, alkylamide sulphonates, alkylarylsulphonates, α-olefinsulphonates, paraffinsulphonates; alkyl sulphosuccinates, alkyl ether sulphosuccinates, alkylamidesulphosuccinates; alkyl sulphoacetates; acylsarcosinates; acylglutamates for which the alkyl and acyl groups comprise from 6 to 24 carbon atoms and the aryl group denotes a phenyl or benzyl group, C₆-C₂₄ alkyl esters of polyglycoside-carboxylic acids, alkyl sulphosuccinamates, acylisethionates and N-acyltaurates, the alkyl or acyl group of which comprises from 12 to 20 carbon atoms; acyllactylates, the acyl group of which comprises from 8 to 20 carbon atoms, and their mixtures.

8. Composition according to any one of Claims 1 to 7, where the synthetic anionic surfactant or surfactants are chosen from alkyl-D-galactoside uronic acids; polyoxyalkylenated (C₆-C₂₄) alkyl ether carboxylic acids, polyoxyalkylenated (C₆-C₂₄) alkylaryl (C₆-C₂₄) ether carboxylic acids, polyoxyalkylenated (C₆-C₂₄) alkylamido ether carboxylic acids, and their salts.

9. Composition according to either of Claims 7 and 8, where the salts are chosen from alkali metal, alkaline earth metal, ammonium, amine or aminoalcohol salts.

10. Composition according to any one of Claims 1 to 9, where the synthetic anionic surfactant or surfactants are chosen from salts of C₆-C₂₄ alkyl ether sulphates comprising from 1 to 30 ethylene oxide groups.

11. Composition according to Claim 9 or 10, where the synthetic anionic surfactant is chosen from sodium (C₁₂-C₁₄) alkyl ether sulphates having a mean number of ethylene oxide groups of between 1 and 4.

12. Composition according to Claim 11, where the synthetic anionic surfactant is sodium laureth sulphate.

13. Composition according to any one of Claims 1 to 12, where the synthetic anionic surfactant or surfactants are present in amounts ranging from 0.1 to 10% by weight as active material and more preferably from 0.5 to 5% by weight, with respect to the total weight of the composition.

14. Composition according to any one of Claims 1 to 13, where the amphoteric surfactant is chosen from aliphatic secondary or tertiary amine derivatives in which the aliphatic group is a linear or branched chain comprising from 8 to 22 carbon atoms and comprising at least one water-solublizing anionic group chosen from carboxylate, sulphonate, sulphate, phosphate or phosphonate; (C₈-C₂₀)alkyl betaines, sulphobetaines, (C₈-C₂₀) alkyl amido (C₆-C₈) alkyl betaines or (C₈-C₂₀) alkyl amido (C₆-C₈) alkyl sulphobetaines, and their mixtures.

15. Composition according to Claim 14, where the amphoteric surfactant is chosen from compounds with the respective structures (1) and (2):
Rₐ-CONHCH₂CH₂-N⁺ (R_{b}) (R_{c}) (CH₂COO-) (1)
in which:
Rₐ represents an alkyl group derived from an acid Rₐ-COOH present in hydrolysed coconut oil or a heptyl, nonyl or undecyl group,
R_{b} represents a β-hydroxyethyl group, and
R_{c} represents a carboxymethyl group;
and
Rₐ' -CONHCH₂CH₂-N (B) (C) (2)
in which:
B represents -CH₂CH₂OX',
C represents-(CH₂)₂-Y', where z = 1 or 2,
X' represents the -CH₂CH₂-COOH group or a hydrogen atom,
Y' represents -COOH or the -CH₂-CHOH-SO₃H group,
Rₐ' represents an alkyl group of an acid Rₐ'-COOH present in hydrolysed coconut oil or linseed oil, an alkyl group, in particular a C₁₇ alkyl group, and its iso form, or an unsaturated C₁₇ group.

16. Composition according to Claim 15, where the amphoteric surfactant is chosen from disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid or cocoamphodipropionic acid.

17. Composition according to Claim 16, where the amphoteric surfactant or surfactants are chosen from (C₈-C₂₀)alkyl betaines.

18. Composition according to Claim 17, where the amphoteric surfactant is cocobetaine.

19. Composition according to any one of Claims 1 to 18, where the amphoteric surfactant or surfactants are present in amounts ranging from 0.1 to 10% by weight as active material and more preferably from 0.5 to 5% by weight, with respect to the total weight of the composition.

20. Composition according to any one of Claims 1 to 19, where the soap(s)/anionic surfactant ratio by weight is greater than 4:1.

21. Composition according to any one of Claims 1 to 20, where the cellulose compounds are chosen from nonionic cellulose ethers.

22. Composition according to Claim 21, where the nonionic cellulose ethers are chosen from alkylcelluloses; hydroxyalkylcelluloses; hydroxyalkyl-alkylcellulose mixed celluloses.

23. Composition according to Claim 21 or 22, where the nonionic cellulose compound is a hydroxypropylcellulose.

24. Composition according to any one of Claims 1 to 23, where the cellulose compound or compounds represent from 0.01 to 3% and more preferably still from 0.05 to 1.5% by weight, with respect to the total weight of the composition.

25. Composition according to any one of Claims 1 to 24, furthermore comprising at least one other additional thickener.

26. Composition according to Claim 25, where the additional thickeners are chosen from:
- electrolytes;
- alkanolamides;
- esters of polyethylene glycol and of mono- or distearic acids;
- polysaccharide biopolymers;
- synthetic polymers;
- clays;
- their mixtures.

27. Composition according to Claim 26, where the additional thickeners are present in concentrations ranging from 0.05 to 4% by weight as active material and more particularly from 0.1 to 3% by weight, with respect to the total weight of the composition.

28. Composition according to any one of Claims 25 to 27, in which the thickening system is a mixture of hydroxypropylmethylcellulose and of alkanolamide.

29. Composition according to Claim 28, in which the thickening system is a hydroxypropylmethylcellulose/cocamide MEA mixture.

30. Composition according to any one of Claims 25 to 29, in which the thickening system is a mixture comprising a hydroxypropylmethylcellulose, an alkanolamide and an electrolyte.

31. Composition according to Claim 30, in which the thickening system is a hydroxypropylmethylcellulose/cocamide MEA/potassium chloride mixture.

32. Composition according to any one of Claims 1 to 31, **characterized in that** the cosmetically acceptable aqueous medium comprises at least one solvent chosen from lower alcohols; polyols; sugars and their mixtures.

33. Composition according to any one of Claims 1 to 32, **characterized in that** it additionally comprises at least one cationic polymer of the polyquaternium type.

34. Composition according to any one of Claims 1 to 33, **characterized in that** it additionally comprises at least one nonionic surfactant.

35. Composition according to any one of Claims 1 to 34, **characterized in that** it additionally comprises at least one adjuvant chosen from oils, active principles, fragrances, preservatives, sequestering agents, pigments, pearlescent agents, inorganic or organic fillers, or dyes.

36. Composition according to Claim 35, where the active principle or principles are chosen from sunscreens; desquamating agents; moisturizing agents; depigmenting agents; propigmenting agents; α-hydroxy acids; antibacterials; agents for combating free radicals; agents for combating pollution; anti-inflammatories; retinoids; algal, fungal, plant, yeast or bacterial extracts; hydrolysed, partially hydrolysed or non-hydrolysed proteins; enzymes, hormones, vitamins and their derivatives, flavonoids and isoflavones, and their mixtures.

37. Cosmetic use of a composition as defined in any one of Claims 1 to 36 as product for cleaning and/or removing make-up from human keratinous substances.

38. Use according to Claim 37, as bath or shower product.

39. Use according to Claim 37, as product for cleaning the hands.

40. Use according to Claim 37, as shampoo.

41. Use according to Claim 37, as make-up-removing product for the eyes, face or lips.

42. Cosmetic process for cleaning soiling residues from human keratinous substances, **characterized in that** a composition as defined in any one of Claims 1 to 36 is applied to the said keratinous substances in the presence of water, **in that** massaging is carried out in order to form a foam and **in that** the foam formed and the soiling residues are removed by rinsing with water.

## Patentansprüche

1. Flüssige reinigende Zusammensetzung auf der Basis von Seifen und synthetischen grenzflächenaktiven Stoffen, **dadurch gekennzeichnet, dass** sie in einem kosmetisch akzeptablen wässrigen Medium enthält:
(a) mindestens eine Fettsäure oder ein Gemisch von Fettsäuren, die vollständig neutralisiert sind, in einer Menge von höchstens 20 Gew.-%, bezogen auf das Gesamtgewicht der Zusammensetzung, wobei die Fettsäure oder die Fettsäuren mit einer anorganischen Base, die unter den Alkalihydroxiden oder Ammoniumhydroxid ausgewählt ist, oder mit einer organischen Base neutralisiert sind, die unter den Aminen und Alkanolaminen ausgewählt ist;
(b) mindestens einen anionischen synthetischen grenzflächenaktiven Stoff;
(c) mindestens einen amphoteren grenzflächenaktiven Stoff; wobei die Gesamtmenge des synthetischen anionischen grenzflächenaktiven Stoffes und des amphoteren grenzflächenaktiven Stoffes als wirksame Substanz ausgedrückt höchstens 10 Gew.-%, bezogen auf das Gesamtgewicht der Zusammensetzung, beträgt;
(d) mindestens ein Verdickungsmittel vom Typ einer nichtionischen Celluloseverbindung.

2. Zusammensetzung nach Anspruch 1, **dadurch gekennzeichnet, dass** die Fettsäure eine Carbonsäure ist, die eine geradkettige oder verzweigte, gesättigte oder ungesättigte Alkylkette mit 6 bis 30 Kohlenstoffatomen, vorzugsweise 12 bis 22 Kohlenstoffatomen aufweist.

3. Zusammensetzung nach Anspruch 2, **dadurch gekennzeichnet, dass** die Fettsäure unter Laurinsäure, Myristinsäure, Palmitinsäure, Stearinsäure und deren Gemischen ausgewählt ist.

4. Zusammensetzung nach Anspruch 3, **dadurch gekennzeichnet, dass** sie ein Gemisch von Fettsäuren enthält, das aus Laurinsäure, Myristinsäure, Palmitinsäure und Stearinsäure besteht.

5. Zusammensetzung nach einem der Ansprüche 1 bis 4, wobei die Base Kaliumhydroxid ist.

6. Zusammensetzung nach einem der Ansprüche 1 bis 5, wobei die Fettsäure(n) in Mengenanteilen von 1 bis 20 Gew.-% und vorzugsweise 5 bis 10 Gew.-%, bezogen auf das Gesamtgewicht der Zusammensetzung, enthalten sind.

7. Zusammensetzung nach einem der Ansprüche 1 bis 6, wobei der oder die synthetische(n) anionische(n) grenzflächenaktive(n) Stoff(e) unter den Salzen der folgenden Verbindungen ausgewählt sind: Alkylsulfate, Alkylethersulfate, Alkylamidoethersulfate, Alkylarylpolyethersulfate, Monoglyceridsulfate; Alkylsulfonate, Alkylamidsulfonate, Alkylarylsulfonate, α-Olefinsulfonate, Paraffinsulfonate; Alkylsulfosuccinate, Alkylethersulfosuccinate, Alkylamidsulfosuccinate; Alkylsulfoacetate; Acylsarcosinate; und Acylglutamate, wobei die Alkyl- und Acylgruppen der Verbindungen 6 bis 24 Kohlenstoffatome aufweisen und die Arylgruppe Phenyl oder Benzyl bedeutet, C₆-₂₄-Alkylester von Polyglycosid-carbonsäuren, Alkylsulfosuccinamate, Acylisethionate und N-Acyltaurate, deren Alkyl- oder Acylgruppe 12 bis 20 Kohlenstoffatome aufweist; Acyllactylate, deren Acylgruppe 8 bis 20 Kohlenstoffatome enthält, und deren Gemische.

8. Zusammensetzung nach einem der Ansprüche 1 bis 7, wobei der oder die synthetische(n) anionische(n) grenzflächenaktive(n) Stoff(e) unter den Alkyl-D-galactosiduronsäuren, polyalkoxylierten Alkyl(C₆₋₂₄)ethercarbonsäuren, polyalkoxylierten Alkyl(C₆₋₂₄)-aryl(C₆₋₂₄)ethercarbonsäuren, polyalkoxylierten Alkyl(C₆₋₂₄)-amidoethercarbonsäuren und deren Salzen ausgewählt sind.

9. Zusammensetzung nach einem der Ansprüche 7 und 8, wobei die Salze unter den Alkalimetallsalzen, Erdalkalimetallsalzen, Ammoniumsalzen, Aminsalzen oder Aminoalkoholsalzen ausgewählt sind.

10. Zusammensetzung nach einem der Ansprüche 1 bis 9, wobei der synthetische (n) anionische(n) grenzflächenaktive(n) Stoff(e) unter den Salzen von C₆₋₂₄-Alkylethersulfaten mit 1 bis 30 Ethylenoxidgruppen ausgewählt sind.

11. Zusammensetzung nach Anspruch 9 oder 10, wobei der synthetische anionische grenzflächenaktive Stoff unter den Natrium-alkyl(C₁₂₋₁₄)ethersulfaten mit einer mittleren Zahl der Ethylenoxidgruppen von 1 bis 4 ausgewählt ist.

12. Zusammensetzung nach Anspruch 11, wobei der synthetische anionische grenzflächenaktive Stoff das Sodium Laureth Sulfate ist.

13. Zusammensetzung nach einem der Ansprüche 1 bis 12, wobei der oder die synthetische(n) anionische(n) grenzflächenaktive(n) Stoff(e) in Mengenanteilen von 0,1 bis 10 Gew.-% wirksame Substanz und insbesondere 0,5 bis 5 Gew.-%, bezogen auf das Gesamtgewicht der Zusammensetzung, enthalten ist.

14. Zusammensetzung nach einem der Ansprüche 1 bis 13, wobei der amphotere grenzflächenaktive Stoff unter den aliphatischen, sekundären oder tertiären Aminderivaten, worin die aliphatische Gruppe eine gerade oder verzweigte Kette mit 8 bis 22 Kohlenstoffatomen ist, die mindestens eine wasserlösliche anionische Gruppe enthält, die unter Carboxylat, Sulfonat, Sulfat, Phosphat oder Phosphonat ausgewählt ist; Alkyl(C₈₋₂₀)betainen, Sulfobetainen, Alkyl(C₈₋₂₀)amidoalkyl(C₆₋₈)betainen oder Alkyl-(C₈₋₂₀)amidoalkyl(C₆₋₈)sulfobetainen und deren Gemischen ausgewählt ist.

15. Zusammensetzung nach Anspruch 14, wobei der amphotere grenzflächenaktive Stoff unter den Verbindungen der folgenden Strukturen (1) und (2) ausgewählt ist:
Rₐ-CONHCH₂CH₂-N(R_{b})(R_{c})(CH₂COO-) (1)
worin bedeuten:
Rₐ eine Alkylgruppe, die von einer Säure Ra-COOH abgeleitet ist, die in hydrolysiertem Kopraöl vorliegt, Heptyl, Nonyl oder Undecyl,
R_{b} β-Hydroxyethyl, und
R_{c} Carboxymethyl;
und
Rₐ'-CONHCH₂CH₂-N(B)(C) (2)
worin bedeuten:
B -CH₂CH₂OX',
C -(CH₂)_{z})-Y' mit z = 1 oder 2,
X' die Gruppe -CH₂CH₂-COOH oder ein Wasserstoffatom,
Y' -COOH oder die Gruppe -CH₂-CHOH-SO₃H,
Ra' eine Alkylgruppe einer Säure Rₐ'-COOH, die in hydrolysiertem Leinöl oder Kopraöl vorliegt, eine Alkylgruppe, insbesondere mit C₁₇ und ihre Isoform, eine ungesättigte C₁₇-Gruppe.

16. Zusammensetzung nach Anspruch 15, wobei der amphotere grenzflächenaktive Stoff unter Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic Acid, Cocoamphodipropionic Acid ausgewählt ist.

17. Zusammensetzung nach Anspruch 16, wobei der oder die amphotere(n) grenzflächenaktive(n) Stoff(e) unter den Alkyl(C₈₋₂₀)betainen ausgewählt sind.

18. Zusammensetzung nach Anspruch 16, wobei der amphotere grenzflächenaktive Stoff das Cocobetain ist.

19. Zusammensetzung nach einem der Ansprüche 1 bis 18, wobei der oder die amphoter(n) grenzflächenaktive(n) Stoff(e) in Mengenanteilen von 0,1 bis 10 Gew.-% wirksame Substanz und vorzugsweise 0,5 bis 5 Gew.-%, bezogen auf das Gesamtgewicht der Zusammensetzung, enthalten sind.

20. Zusammensetzung nach einem der Ansprüche 1 bis 19, wobei das Gewichtsverhältnis Seife(n)/anionischer grenzflächenaktiver Stoff größer als 4:1 ist.

21. Zusammensetzung nach einem der Ansprüche 1 bis 20, wobei die Celluloseverbindungen unter den nichtionischen Celluloseethern ausgewählt sind.

22. Zusammensetzung nach Anspruch 21, wobei die nichtionischen Celluloseether unter den Alkylcellulosen; Hydroxyalkylcellulosen; gemischten Hydroxyalkyl-alkylcellulosen ausgewählt sind.

23. Zusammensetzung nach Anspruch 21 oder 22, wobei die nichtionische Celluloseverbindung eine Hydroxypropylcellulose ist.

24. Zusammensetzung nach einem der Ansprüche 1 bis 23, wobei die Celluloseverbindung(en) 0,01 bis 3 % und noch bevorzugter 0,05 bis 1,5 Gew.-%, bezogen auf das Gesamtgewicht der Zusammensetzung, aufmachen.

25. Zusammensetzung nach einem der Ansprüche 1 bis 24, die ferner mindestens ein ergänzendes weiteres Verdickungsmittel enthält.

26. Zusammensetzung nach Anspruch 25, wobei die ergänzenden Verdickungsmittel ausgewählt sind unter:
- Elektrolyten;
- Alkanolamiden;
- Monostearinsäureestern oder Distearinsäresestern von Polyethylenglycol;
- Polysaccharid-Biopolymeren;
- synthetischen Polymeren;
- Tonen; und
- deren Gemischen.

27. Zusammensetzung nach Anspruch 26, wobei die ergänzenden Verdickungsmittel in Konzentrationen von 0,05 bis 4 Gew.-% wirksame Substanz, bezogen auf das Gesamtgewicht der Zusammensetzung, und insbesondere 0,1 bis 3 Gew.-% enthalten sind.

28. Zusammensetzung nach einem der Ansprüche 25 bis 27, wobei das Verdickungssystem ein Gemisch aus Hydroxypropylmethylcellulose und Alkanolamid ist.

29. Zusammensetzung nach Anspruch 28, wobei das Verdickungssystem ein Gemisch aus Hydroxypropylmethylcellulose und Cocamide MEA ist.

30. Zusammensetzung nach einem der Ansprüche 25 bis 29, wobei das Verdickungssystem ein Gemisch ist, das eine Hydroxypropylmethylcellulose, ein Alkanolamid und einen Elektrolyten enthält

31. Zusammensetzung nach Anspruch 30, wobei das Verdickungssystem ein Gemisch aus Hydroxypropylmethylcellulose/Cocamide MEA/Kaliumchlorid ist.

32. Zusammensetzung nach einem der Ansprüche 1 bis 31, **dadurch gekennzeichnet, dass** das kosmetisch akzeptable wässrige Medium mindestens ein Lösungsmittel enthält, das unter den niederen Alkoholen; Polyolen; Zuckern und deren Gemischen ausgewählt ist.

33. Zusammensetzung nach einem Ansprüche 1 bis 32, **dadurch gekennzeichnet, dass** sie ferner mindestens ein kationisches Polymer vom Polyquaterniumtyp enthält.

34. Zusammensetzung nach einem der Ansprüche 1 bis 33, **dadurch gekennzeichnet, dass** sie ferner mindestens einen nichtionischen grenzflächenaktiven Stoff enthält.

35. Zusammensetzung nach einem der Ansprüche 1 bis 34, **dadurch gekennzeichnet, dass** sie ferner mindestens einen Zusatzstoff enthält, der unter den Ölen, Wirkstoffen, Parfums, Konservierungsmitteln, Maskierungsmitteln, Pigmenten, Perlglanzpigmenten, anorganischen oder organischen Füllstoffen und Farbmitteln ausgewählt ist.

36. Zusammensetzung nach Anspruch 35, wobei der Wirkstoff oder die Wirkstoffe unter den Sonnenschutzfiltem; abschuppenden Wirkstoffen; Hydratisierungsmitteln; depigmentierenden Wirkstoffen; pigmentierungsfördernden Wirkstoffen; α-Hydroxysäuren, antibakteriellen Wirkstoffen; Radikalfängern für freie Radikale; Anti-Pollutions-Wirkstoffen; entzündungshemmenden Wirkstoffen; Retinoiden; Algenextrakten, Pilzextrakten, Pflanzenextrakten, Hefeextrakten und Bakterienextrakten; hydrolysierten Proteinen, teilweise hydrolysierten Proteinen oder nicht hydrolysierten Proteinen; Enzymen, Hormonen, Vitaminen und deren Derivaten, Flavonoiden und Isoflavonen, und deren Gemischen ausgewählt sind.

37. Kosmetische Verwendung einer Zusammensetzung nach einem der Ansprüche 1 bis 36 als Produkt zur Reinigung und/oder zum Abschminken von menschlichen Keratinsubstanzen.

38. Verwendung nach Anspruch 37 als Produkt für das Bad oder die Dusche.

39. Verwendung nach Anspruch 37 als Produkt zur Reinigung der Hände.

40. Verwendung nach Anspruch 37 als Haarwaschmittel.

41. Verwendung nach Anspruch 37 als abschminkendes Produkt für die Augen, das Gesicht oder die Lippen.

42. Kosmetisches Verfahren zur Reinigung von Verschmutzungen an menschlichen Keratinsubstanzen, **dadurch gekennzeichnet, dass** eine Zusammensetzung nach einem der Ansprüche 1 bis 36 auf die Keratinsubstanzen in Gegenwart von Wasser aufgetragen wird, zur Bildung eines Schaums massiert wird und der gebildete Schaum und die Verschmutzungen durch Spülen mit Wasser entfernt werden.
